(19) Europäisches Patentamt
European Patent Office
Office européen des brevets

(11) **EP 3 936 538 A1**

(12) **EUROPEAN PATENT APPLICATION**
published in accordance with Art. 153(4) EPC

(43) Date of publication:
**12.01.2022 Bulletin 2022/02**

(51) Int Cl.:
**C08F 20/06** (2006.01)   **C08J 3/24** (2006.01)
**A61F 13/53** (2006.01)   **A61F 13/84** (2006.01)
**A61L 15/60** (2006.01)

(21) Application number: **20769580.0**

(22) Date of filing: **05.03.2020**

(86) International application number:
**PCT/JP2020/009508**

(87) International publication number:
**WO 2020/184395 (17.09.2020 Gazette 2020/38)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **08.03.2019  JP 2019043017**

(71) Applicant: **Sumitomo Seika Chemicals Co., Ltd.
Kako-gun, Hyogo 675-0145 (JP)**

(72) Inventor: **ITO Takashi
Himeji-shi, Hyogo 672-8076 (JP)**

(74) Representative: **Nederlandsch Octrooibureau
P.O. Box 29720
2502 LS The Hague (NL)**

(54) **WATER ABSORBING RESIN PARTICLES AND METHOD FOR PRODUCING SAME, ABSORBENT BODY. ABSORBENT ARTICLE, AND METHOD FOR ADJUSTING PERMEATION SPEED**

(57)    Water-absorbent resin particles 10a, in which a gel flow rate measured by the following procedures having (1) to (3) is 10 to 70 g/min:
(1) mixing 1 part by mass of the water-absorbent resin particles and 199 parts by mass of water to obtain a gel,
(2) accommodating the gel in a funnel in a state where a circular lower end opening with a diameter of 8 mm in the funnel is closed, and
(3) measuring an outflow amount of the gel per unit time after a lapse of 3 minutes from opening of the lower end opening as the gel flow rate.

**Fig.1**

EP 3 936 538 A1

## Description

## Technical Field

The present invention relates to water-absorbent resin particles and a method for producing the same, an absorber, an absorbent article, and a method for adjusting a permeation rate.

## Background Art

In the related art, an absorber containing water-absorbent resin particles has been used in an absorbent article for absorbing a liquid (for example, urine) having water as a main component. For example, Patent Literature 1 below discloses water-absorbent resin particles having a particle diameter that is suitably used for absorbent articles such as diapers. In addition, Patent Literature 2 discloses a method of using a hydrogel-absorbent polymer having specific saline flow conductivity, performance under pressure, and the like as an effective absorbent member for storing a body fluid such as urine.

## Citation List

## Patent Literature

[Patent Literature 1] Japanese Unexamined Patent Publication No. H06-345819
[Patent Literature 2] Published Japanese Translation No. H09-510889 of the PCT International Publication

## Summary of Invention

## Technical Problem

In a case where a liquid provided to an absorbent article does not sufficiently permeate the absorbent article, there may be a problem of the excess liquid flowing on a surface of the absorbent article, resulting in its leakage to the outside of the absorbent article. Therefore, an absorbent article is required to cause a liquid to permeate it at a better permeation rate.
An object of one aspect of the present invention is to provide water-absorbent resin particles capable of obtaining an absorbent article having a better permeation rate and a method for producing the water-absorbent resin particles, and a method for adjusting a permeation rate. An object of another aspect of the present invention is to provide an absorber and an absorbent article using the water-absorbent resin particles.

## Solution to Problem

One aspect of the present invention provides water-absorbent resin particles in which a gel flow rate measured by the following procedures having (1) to (3) is 10 to 70 g/min:

    (1) mixing 1 part by mass of the water-absorbent resin particles and 199 parts by mass of water to obtain a gel,
    (2) accommodating the gel in a funnel in a state where a circular lower end opening with a diameter of 8 mm in the funnel is closed, and
    (3) measuring an outflow amount of the gel per unit time after a lapse of 3 minutes from opening of the lower end opening as the gel flow rate.

According to the above-mentioned water-absorbent resin particles, it is possible to obtain an absorbent article having a better permeation rate.
Another aspect of the present invention provides an absorber containing the above-mentioned water-absorbent resin particles.
Still another aspect of the present invention provides an absorbent article including the above-mentioned absorber.
Still another aspect of the present invention provides a method for producing water-absorbent resin particles, the method including selecting water-absorbent resin particles based on a gel flow rate measured by the following procedures having (1) to (3):

(1) mixing 1 part by mass of the water-absorbent resin particles and 199 parts by mass of water to obtain a gel,
(2) accommodating the gel in a funnel in a state where a circular lower end opening with a diameter of 8 mm in the funnel is closed, and
(3) measuring an outflow amount of the gel per unit time after a lapse of 3 minutes from opening of the lower end opening as the gel flow rate.

According to the above-mentioned method for producing water-absorbent resin particles, it is possible to obtain an absorbent article having a better permeation rate by selecting water-absorbent resin particles based on a gel flow rate.

Still another aspect of the present invention provides a method for adjusting a permeation rate in an absorbent article including an absorber, in which the absorber contains water-absorbent resin particles, and the method includes adjusting a gel flow rate measured by the following procedures having (1) to (3):

(1) mixing 1 part by mass of the water-absorbent resin particles and 199 parts by mass of water to obtain a gel,
(2) accommodating the gel in a funnel in a state where a circular lower end opening with a diameter of 8 mm in the funnel is closed, and
(3) measuring an outflow amount of the gel per unit time after a lapse of 3 minutes from opening of the lower end opening as the gel flow rate.

According to the above-mentioned method for adjusting a permeation rate, it is possible to obtain an absorbent article having a better permeation rate by adjusting a gel flow rate.

**Advantageous Effects of Invention**

According to one aspect of the present invention, it is possible to provide water-absorbent resin particles capable of obtaining an absorbent article having a better permeation rate and a method for producing the water-absorbent resin particles, and a method for adjusting a permeation rate. Furthermore, according to another aspect of the present invention, it is possible to provide an absorber and an absorbent article using the water-absorbent resin particles. According to still another aspect of the present invention, it is possible to provide use of water-absorbent resin particles, an absorber, and an absorbent article to absorption of a liquid. According to still another aspect of the present invention, it is possible to provide use of water-absorbent resin particles, an absorber, and an absorbent article to adjustment of a permeation rate in an absorbent article.

**Brief Description of Drawings**

Fig. 1 is a cross-sectional view showing an example of an absorbent article.
Fig. 2 is a plan view showing an outline of a stirring blade used in Examples.
Fig. 3 is a schematic view showing a measurement device for a water absorption amount of water-absorbent resin particles under a load.
Fig. 4 is a schematic view showing a measurement device for fluidity of a gel.

**Description of Embodiments**

Hereinafter, embodiments of the present invention will be described in detail. However, the present invention is not limited to the following embodiments, and can be variously modified and implemented within the scope of the gist thereof.

In the present specification, "acrylic" and "methacrylic" are collectively referred to as "(meth)acrylic". "Acrylate" and "methacrylate" are also referred to as "(meth)acrylate". "(Poly)" means both of a case where there is a prefix of "poly" and a case where there is no prefix thereof. In a numerical value range described in a stepwise manner in the present specification, an upper limit value or a lower limit value of a numerical value range in a certain step can be arbitrarily combined with an upper limit value or a lower limit value of a numerical value range in another step. In a numerical value range described in the present specification, an upper limit value or a lower limit value of the numerical value range may be replaced with a value shown in Examples. "Water-soluble" means that a solubility of 5% by mass or more is exhibited in water at 25°C. For materials exemplified in the present specification, one kind may be used alone, or two or more kinds may be used in combination. In a case where there are a plurality of substances corresponding to each of components in a composition, a content of each of the components in the composition means the total amount of the plurality of substances present in the composition unless otherwise specified. "Physiological saline" refers to an aqueous solution of 0.9% by mass sodium chloride.

In water-absorbent resin particles of the present embodiment, a gel flow rate measured by the following procedures having (1) to (3) is 10 to 70 g/min:

(1) mixing 1 part by mass of the water-absorbent resin particles and 199 parts by mass of water to obtain a gel,
(2) accommodating the gel in a funnel in a state where a circular lower end opening with a diameter of 8 mm in the funnel is closed, and
(3) measuring an outflow amount of the gel per unit time after a lapse of 3 minutes from opening of the lower end opening as the gel flow rate.

According to the water-absorbent resin particles, it is possible to obtain an absorbent article having a better permeation rate. The reason why such an effect is obtained is not clear, but the inventor of the present invention speculates as follows. However, the reason is not limited to the following contents. That is, the water-absorbent resin particles have crosslinking points which are not dissolved to the extent and which do not significantly inhibit swelling to the extent when swollen by absorbing a liquid such as water. Meanwhile, even when crosslinking points that enable appropriate swelling are present, when the crosslinking points are unevenly distributed, swelling of the gel is also biased. In this case, insufficiently swollen portions are generated, and they lead to an unfavorable phenomenon such as gel blocking. That is, regarding the water-absorbent resin particles in which a gel flow rate is a specific value, it is thought that an absorbent article in which a permeation rate is better is obtained since the water-absorbent resin particles are appropriately crosslinked, and crosslinking of the particles is uniform.

The gel flow rate is preferably 15 g/min or more, 20 g/min or more, 25 g/min or more, 30 g/min or more, 35 g/min or more, 40 g/min or more, or 45 g/min or more, from the viewpoint of easily obtaining an better permeation rate in an absorbent article. The gel flow rate is preferably 65 g/min or less, 60 g/min or less, 55 g/min or less, or 50 g/min or less, from the viewpoint of easily obtaining a better permeation rate in an absorbent article. As the gel flow rate, it is possible to use a gel flow rate at room temperature (25°C ± 2°C).

The gel used in the step (1) in the gel flow rate test can be obtained as a swollen gel that is swollen 200 times by mixing 1 part by mass of the water-absorbent resin particles and 199 parts by mass of ion-exchanged water.

The funnel used in the step (2) has a circular lower end opening (discharge port) with the diameter of 8 mm. The lower end opening can be opened and closed by a damper or the like. The funnel has, for example, an upper end opening (supply port) for supplying the gel, and the gel supplied from the upper end opening is discharged from the lower end opening. The funnel has, for example, a tapered shape that narrows from the upper end opening to the lower end opening. The inclination angle of a side wall of the funnel is, for example, 20° with respect to an axial direction of the funnel. The funnel can be installed horizontally. The constituent material of the funnel is, for example, stainless steel.

In the step (3), a receiver that receives the gel is disposed downward in a vertical direction with respect to the funnel, and an outflow amount of the gel after a lapse of 3 minutes from opening of the lower end opening is measured. The gel can be outflowed in a state where an upper end of the receiver is located 38 mm below the discharge port of the funnel. Then, in the step (3), an outflow amount (gel flow rate) of the gel per unit time can be obtained by dividing the outflow amount of the gel at a time point when 3 minutes have elapsed by the elapsed time (3 minutes).

The water-absorbent resin particles of the present embodiment may be any water-absorbent resin particles as long as the water-absorbent resin particles can retain water, and the liquid to be absorbed can contain water. The water-absorbent resin particles of the present embodiment are better in absorbency of a body fluid such as urine, sweat, blood (for example, menstrual blood). The water-absorbent resin particles of the present embodiment can be used as a constituent component of the absorber of the present embodiment.

The water retention amount (water retention amount under no pressurization) of physiological saline of the water-absorbent resin particles of the present embodiment is preferably within the following range. The water retention amount is preferably 10 g/g or more, 15 g/g or more, 20 g/g or more, 25 g/g or more, 30 g/g or more, or 32 g/g or more, from the viewpoint of easily obtaining a better permeation rate in an absorbent article. The water retention amount is preferably 80 g/g or less, less than 80 g/g, 75 g/g or less, 70 g/g or less, 65 g/g or less, 60 g/g or less, 55 g/g or less, 50 g/g or less, 48 g/g or less, 45 g/g or less, 44 g/g or less, 43 g/g or less, 42 g/g or less, or 40 g/g or less, from the viewpoint of easily obtaining a better permeation rate in an absorbent article. From these viewpoints, the water retention amount is preferably 10 to 80 g/g and is more preferably 30 to 60 g/g.

The water absorption amount of physiological saline of the water-absorbent resin particles of the present embodiment under the load of 4.14 kPa is preferably within the following range. The water absorption amount is preferably 10 mL/g or more, 12 mL/g or more, 15 mL/g or more, 17 mL/g or more, 18 mL/g or more, 20 mL/g or more, 22 mL/g or more, or 24 mL/g or more, from the viewpoint of easily obtaining a better permeation rate in an absorbent article. The water absorption amount is preferably 40 mL/g or less, 35 mL/g or less, 30 mL/g or less, or 28 mL/g or less, from the viewpoint of easily inhibiting excessive swelling in an absorbent article. From these viewpoints, the water absorption amount is preferably 10 to 40 mL/g and is more preferably 12 to 35 mL/g. As the water absorption amount, a water absorption amount at room temperature (25°C ± 2°C) can be used. The water absorption amount can be measured by a method described in Examples to be described later.

Examples of the shape of the water-absorbent resin particles of the present embodiment include a substantially spherical shape, a crushed shape, and a granular shape. The median particle diameter of the water-absorbent resin particles (water-absorbent resin particles before absorbing water) of the present embodiment is preferably within the following range. The median particle diameter is preferably 250 μm or more, 280 μm or more, 300 μm or more, 310 μm or more, 320 μm or more, 330 μm or more, 340 μm or more, 350 μm or more, or 360 μm or more, from the viewpoint that a favorable permeation rate of an absorbent article is easily maintained by avoiding gel blocking. The median particle diameter is preferably 600 μm or less, 550 μm or less, 500 μm or less, 450 μm or less, 400 μm or less, 380 μm or less, or 370 μm or less, from the viewpoint of easily keeping the touch feeling of the absorbent article soft. From these viewpoints, the median particle diameter is preferably 250 to 600 μm. The water-absorbent resin particles of the present embodiment may have a desired particle size distribution at the time of being obtained by a production method to be described later, but the particle size distribution may be adjusted by performing an operation such as particle size adjustment using classification with a sieve.

The water-absorbent resin particles of the present embodiment can contain a crosslinking polymer (a crosslinking polymer having a structural unit derived from an ethylenically unsaturated monomer) obtained by polymerizing a monomer containing an ethylenically unsaturated monomer, as polymer particles, for example. That is, the water-absorbent resin particles of the present embodiment can have a structural unit derived from an ethylenically unsaturated monomer, and can contain polymer particles including a crosslinking polymer having a structural unit derived from an ethylenically unsaturated monomer. As the ethylenically unsaturated monomer, a water-soluble ethylenically unsaturated monomer can be used. Examples of the polymerization method include a reverse phase suspension polymerization method, an aqueous solution polymerization method, a bulk polymerization method, and a precipitation polymerization method. Among these, the reverse phase suspension polymerization method or the aqueous solution polymerization method is preferable from the viewpoint of ensuring good water-absorbent characteristics (such as a water retention amount) of the obtained water-absorbent resin particles and facilitating control of the polymerization reaction. In the following, as a method for polymerizing an ethylenically unsaturated monomer, a reverse phase suspension polymerization method will be described as an example.

The ethylenically unsaturated monomer is preferably water-soluble, and examples thereof include (meth)acrylic acid and a salt thereof, 2-(meth)acrylamide-2-methylpropanesulfonic acid and a salt thereof, (meth)acrylamide, N,N-dimethyl (meth)acrylamide, 2-hydroxyethyl (meth)acrylate, N-methylol (meth)acrylamide, polyethylene glycol mono(meth)acrylate, N,N-diethylaminoethyl (meth)acrylate, N,N-diethylaminopropyl (meth)acrylate, and diethylaminopropyl (meth)acrylamide. In a case where the ethylenically unsaturated monomer has an amino group, the amino group may be quaternized. The ethylenically unsaturated monomer may be used alone, or may be used in combination of two or more kinds thereof. The functional group, such as a carboxyl group and an amino group, of the above-mentioned monomer can function as a functional group capable of crosslinking in a surface crosslinking step to be described later.

Among these, from the viewpoint of industrial availability, the ethylenically unsaturated monomer preferably contains at least one compound selected from the group consisting of (meth)acrylic acid and a salt thereof, acrylamide, methacrylamide, and N,N-dimethyl acrylamide, and more preferably contains at least one compound selected from the group consisting of (meth)acrylic acid and a salt thereof, and acrylamide. From the viewpoint of further enhancing water-absorbent characteristics (such as a water retention amount), the ethylenically unsaturated monomer further more preferably contains at least one compound selected from the group consisting of (meth)acrylic acid and a salt thereof. That is, the water-absorbent resin particles preferably have a structural unit derived from at least one selected from the group consisting of (meth)acrylic acid and a salt thereof.

As the monomer for obtaining the water-absorbent resin particles, a monomer other than the above-mentioned ethylenically unsaturated monomer may be used. Such a monomer can be used by being mixed with an aqueous solution containing the above-mentioned ethylenically unsaturated monomer, for example. The use amount of the ethylenically unsaturated monomer is preferably 70 to 100 mol% with respect to the total amount of the monomer (the total amount of the monomer for obtaining the water-absorbent resin particles. For example, the total amount of the monomers that provide a structural unit of the crosslinking polymer. The same applies hereinafter). Among these, the ratio of (meth)acrylic acid and a salt thereof is more preferably 70 to 100 mol% with respect to the total amount of the monomers. "Ratio of (meth)acrylic acid and a salt thereof" means the ratio of the total amount of (meth)acrylic acid and a salt thereof.

According to the present embodiment, as an example of the water-absorbent resin particles, it is possible to provide the water-absorbent resin particle containing a crosslinking polymer having a structural unit derived from an ethylenically unsaturated monomer, in which the ethylenically unsaturated monomer contains at least one compound selected from the group consisting of (meth)acrylic acid and a salt thereof, and the ratio of (meth)acrylic acid and a salt thereof is 70 to 100 mol% with respect to the total amount of the monomer for obtaining the water-absorbent resin particles (for example, the total amount of the monomer that provides a structural unit of the crosslinking polymer).

The ethylenically unsaturated monomer is usually preferably used as an aqueous solution. The concentration of the ethylenically unsaturated monomer in the aqueous solution containing the ethylenically unsaturated monomer (hereinafter, simply referred to as "monomer aqueous solution") is preferably 20% by mass or more and a saturated concentration or less, more preferably 25 to 70% by mass, and further more preferably 30 to 55% by mass. Examples of the water used in the aqueous solution include tap water, distilled water, and ion-exchanged water.

In a case where the ethylenically unsaturated monomer has an acid group, the monomer aqueous solution may be used by neutralizing the acid group with an alkaline neutralizing agent. The degree of neutralization of the ethylenically unsaturated monomer by the alkaline neutralizing agent is preferably 10 to 100 mol%, more preferably 50 to 90 mol%, and further more preferably 60 to 80 mol% of the acid group in the ethylenically unsaturated monomer, from the viewpoint of increasing an osmotic pressure of the obtained water-absorbent resin particles, and further enhancing water-absorbent characteristics (such as a water retention amount). Examples of the alkaline neutralizing agent include alkali metal salts such as sodium hydroxide, sodium carbonate, sodium hydrogen carbonate, potassium hydroxide, and potassium carbonate; and ammonia. The alkaline neutralizing agent may be used alone, or may be used in combination of two or more kinds thereof. The alkaline neutralizing agent may be used in the form of an aqueous solution to simplify the neutralization operation. Neutralization of the acid group of the ethylenically unsaturated monomer can be performed by adding an aqueous solution of sodium hydroxide, potassium hydroxide, or the like dropwise in the above-mentioned monomer aqueous solution and mixing therewith.

In a reverse phase suspension polymerization method, a monomer aqueous solution is dispersed in a hydrocarbon dispersion medium in the presence of a surfactant, and polymerization of the ethylenically unsaturated monomer can be performed using a radical polymerization initiator or the like. As the radical polymerization initiator, a water-soluble radical polymerization initiator can be used.

Examples of the surfactant include a nonionic surfactant and an anionic surfactant. Examples of the nonionic surfactant include sorbitan fatty acid esters, polyglycerin fatty acid esters, sucrose fatty acid esters, polyoxyethylene sorbitan fatty acid esters, polyoxyethylene glycerin fatty acid esters, sorbitol fatty acid esters, polyoxyethylene sorbitol fatty acid esters, polyoxyethylene alkyl ethers, polyoxyethylene alkylphenyl ethers, polyoxyethylene castor oil, polyoxyethylene hydrogenated castor oil, alkylallyl formaldehyde condensed polyoxyethylene ethers, polyoxyethylene polyoxypropylene block copolymers, polyoxyethylene polyoxypropyl alkyl ethers, and polyethylene glycol fatty acid esters. Examples of the anionic surfactant include fatty acid salts, alkylbenzene sulfonate, alkylmethyl taurate, polyoxyethylene alkylphenyl ether sulfuric acid ester salts, polyoxyethylene alkyl ether sulfonic acid salts, phosphoric acid esters of polyoxyethylene alkyl ethers, and phosphoric acid esters of polyoxyethylene alkyl allyl ethers. The surfactant may be used alone, or may be used in combination of two or more kinds thereof.

From the viewpoint of a good state of the W/O type reverse phase suspension, easily obtaining water-absorbent resin particles having a suitable particle diameter, and industrial availability, the surfactant preferably contains at least one compound selected from the group consisting of sorbitan fatty acid esters, polyglycerin fatty acid esters, and sucrose fatty acid esters. From the viewpoint of easily obtaining an appropriate particle size distribution of the water-absorbent resin particles, and from the viewpoint of easily improving water-absorbent characteristics (such as a water retention amount) of the water-absorbent resin particles and performances of the absorbent article using the same, the surfactant preferably contains sucrose fatty acid ester, and more preferably is sucrose stearic acid ester.

The use amount of the surfactant is preferably 0.05 to 10 parts by mass, more preferably 0.08 to 5 parts by mass, and further more preferably 0.1 to 3 parts by mass with respect to 100 parts by mass of the monomer aqueous solution, from the viewpoint of obtaining a sufficient effect on the use amount and economic efficiency.

In the reverse phase suspension polymerization, a polymeric dispersant may be used in combination with the above-mentioned surfactant. Examples of the polymeric dispersant include maleic anhydride-modified polyethylene, maleic anhydride-modified polypropylene, a maleic anhydride-modified ethylene-propylene copolymer, a maleic anhydride-modified EPDM (ethylene propylene diene terpolymer), maleic anhydride-modified polybutadiene, a maleic anhydride-ethylene copolymer, a maleic anhydride-propylene copolymer, a maleic anhydride-ethylene-propylene copolymer, a maleic anhydride-butadiene copolymer, polyethylene, polypropylene, an ethylene-propylene copolymer, oxidized polyethylene, oxidized polypropylene, an oxidized ethylene-propylene copolymer, an ethylene-acrylic acid copolymer, ethyl cellulose, and ethyl hydroxyethyl cellulose. The polymeric dispersant may be used alone or may be used in combination of two or more kinds thereof. From the viewpoint of better dispersion stability of the monomer, the polymeric dispersant is preferably at least one selected from the group consisting of maleic anhydride-modified polyethylene, maleic anhydride-modified polypropylene, a maleic anhydride-modified ethylene-propylene copolymer, a maleic anhydride-ethylene copolymer, a maleic anhydride-propylene copolymer, a maleic anhydride-ethylene-propylene copolymer, polyethylene, polypropylene, an ethylene-propylene copolymer, oxidized polyethylene, oxidized polypropylene, and an oxidized ethylene-propylene copolymer.

The use amount of the polymeric dispersant is preferably 0.05 to 10 parts by mass, more preferably 0.08 to 5 parts by mass, and further more preferably 0.1 to 3 parts by mass with respect to 100 parts by mass of the monomer aqueous solution, from the viewpoint of obtaining a sufficient effect on the use amount and economic efficiency.

The hydrocarbon dispersion medium may contain at least one compound selected from the group consisting of chain aliphatic hydrocarbons having 6 to 8 carbon atoms and alicyclic hydrocarbons having 6 to 8 carbon atoms. Examples of the hydrocarbon dispersion medium include chain aliphatic hydrocarbons such as n-hexane, n-heptane, 2-methylhexane, 3-methylhexane, 2,3-dimethylpentane, 3-ethylpentane, and n-octane; alicyclic hydrocarbons such as cyclohexane, methylcyclohexane, cyclopentane, methylcyclopentane, trans-1,2-dimethylcyclopentane, cis-1,3-dimethylcyclopentane, and trans-1,3-dimethylcyclopentane; and aromatic hydrocarbons such as benzene, toluene, and xylene. The hydrocarbon dispersion medium may be used alone, or may be used in combination of two or more kinds thereof.

The hydrocarbon dispersion medium may contain at least one selected from the group consisting of n-heptane and cyclohexane from the viewpoint of industrial availability and stable quality. In addition, from the same viewpoint, as the mixture of the above-mentioned hydrocarbon dispersion medium, for example, a commercially available Exxsol Heptane (manufactured by ExxonMobil: containing 75% to 85% of n-heptane and isomeric hydrocarbons) may be used.

The use amount of the hydrocarbon dispersion medium is preferably 30 to 1000 parts by mass, more preferably 40 to 500 parts by mass, and further more preferably 50 to 400 parts by mass with respect to 100 parts by mass of the monomer aqueous solution, from the viewpoint of appropriately removing the heat of polymerization and easily controlling the polymerization temperature. In a case where the use amount of the hydrocarbon dispersion medium is 30 parts by mass or more, the polymerization temperature tends to be easily controlled. In a case where the use amount of the hydrocarbon dispersion medium is 1000 parts by mass or less, the productivity of polymerization tends to be improved, which is economical.

The radical polymerization initiator is preferably water-soluble, and examples thereof include persulfates such as potassium persulfate, ammonium persulfate, and sodium persulfate; peroxides such as methyl ethyl ketone peroxide, methyl isobutyl ketone peroxide, di-t-butyl peroxide, t-butyl cumyl peroxide, t-butyl peroxyacetate, t-butyl peroxyisobutyrate, t-butyl peroxypivalate, and hydrogen peroxide; and azo compounds such as 2,2'-azobis(2-amidinopropane) dihydrochloride, 2,2'-azobis[2-(N-phenylamidino)propane] dihydrochloride, 2,2'-azobis[2-(N-allylamidino)propane] dihydrochloride, 2,2'-azobis[2-(2-imidazolin-2-yl)propane] dihydrochloride, 2,2'-azobis{2-[1-(2-hydroxyethyl)-2-imidazolin-2-yl]propane} dihydrochloride, 2,2'-azobis {2-methyl-N- [1,1-bis(hydroxymethyl)-2-hydroxyethyl]propionamide}, 2,2'-azobis[2-methyl-N-(2-hydroxyethyl)-propionamide], and 4,4'-azobis(4-cyanovaleric acid). The radical polymerization initiator may be used alone, or may be used in combination of two or more kinds thereof. The radical polymerization initiator is preferably at least one selected from the group consisting of potassium persulfate, ammonium persulfate, sodium persulfate, 2,2'-azobis(2-amidinopropane) dihydrochloride, 2,2'-azobis[2-(2-imidazolin-2-yl)propane] dihydrochloride, and 2,2'-azobis {2-[1-(2-hydroxyethyl)-2-imidazolin-2-yl]propane} dihydrochloride; and is more preferably at least one azo compound selected from the group consisting of 2,2'-azobis(2-amidinopropane) dihydrochloride, 2,2'-azobis[2-(2-imidazolin-2-yl)propane] dihydrochloride, and 2,2'-azobis{2-[1-(2-hydroxyethyl)-2-imidazolin-2-yl]propane} dihydrochloride. The proportion of the azo compound is preferably 20 to 100 mol%, more preferably 35 to 90 mol%, and further more preferably 50 to 85 mol% with respect to the total amount of the radical polymerization initiator.

The use amount of the radical polymerization initiator may be 0.05 to 10 mmol with respect to 1 mol of the ethylenically unsaturated monomer. In a case where the use amount of the radical polymerization initiator is 0.05 mmol or more, the polymerization reaction does not require a long time and is efficient. In a case where the use amount of the radical polymerization initiator is 10 mmol or less, the occurrence of a rapid polymerization reaction is easily inhibited.

The above-mentioned radical polymerization initiator can also be used as a redox polymerization initiator in combination with a reducing agent such as sodium sulfite, sodium hydrogen sulfite, ferrous sulfate, and L-ascorbic acid.

At the time of the polymerization reaction, the monomer aqueous solution used for the polymerization may contain a chain transfer agent. Examples of the chain transfer agent include hypophosphites, thiols, thiolic acids, secondary alcohols, and amines.

The monomer aqueous solution used for the polymerization may contain a thickener in order to control the particle diameter of the water-absorbent resin particles. Examples of the thickener include hydroxyethyl cellulose, hydroxypropyl cellulose, methyl cellulose, carboxymethyl cellulose, polyethylene glycol, polyacrylamide, polyethyleneimine, dextrin, sodium alginate, polyvinyl alcohol, polyvinylpyrrolidone, and polyethylene oxide. In a case where the stirring speed at the time of polymerization is the same, the higher the viscosity of the monomer aqueous solution, the larger the median particle diameter of the obtained particles tends to be.

Crosslinking by self-crosslinking may occur during polymerization, but crosslinking may be performed by using an internal crosslinking agent. In a case where an internal crosslinking agent is used, water-absorbent characteristics (such as a water retention amount) of the water-absorbent resin particles are easily controlled. The internal crosslinking agent is usually added to a reaction solution during the polymerization reaction. Examples of the internal crosslinking agent include di or tri (meth)acrylic acid esters of polyols such as ethylene glycol, propylene glycol, trimethylolpropane, glycerin, polyoxyethylene glycol, polyoxypropylene glycol, and polyglycerin; unsaturated polyesters obtained by reacting the above-mentioned polyols with unsaturated acids (such as maleic acid and fumaric acid); bis(meth)acrylamides such as N,N'-methylenebis(meth)acrylamide; di or tri (meth)acrylic acid esters obtained by reacting a polyepoxide with (meth)acrylic acid; carbamyl di(meth)acrylate esters obtained by reacting a polyisocyanate (such as tolylene diisocyanate and hexamethylene diisocyanate) with hydroxyethyl (meth)acrylate; compounds having two or more polymerizable unsaturated groups, such as allylated starch, allylated cellulose, diallyl phthalate, N, N', N"-triallyl isocyanurate, and divinylbenzene; polyglycidyl compounds such as (poly)ethylene glycol diglycidyl ether, (poly)propylene glycol diglycidyl ether, (poly)glycerin diglycidyl ether, (poly)glycerin triglycidyl ether, (poly)propylene glycol polyglycidyl ether, and polyglycerol polyglycidyl ether; haloepoxy compounds such as epichlorohydrin, epibromohydrin, and $\alpha$-methyl epichlorohydrin; and compounds having two or more reactive functional groups, such as isocyanate compounds (such as 2,4-tolylene diisocyanate and hexamethylene diisocyanate). The internal crosslinking agent may be used alone, or may be used in combination of two or more kinds thereof. The internal crosslinking agent is preferably a polyglycidyl compound, is more preferably a diglycidyl ether compound, and is further more preferably at least one selected from the group consisting of (poly)ethylene glycol diglycidyl ether, (poly)propylene glycol diglycidyl ether, and (poly)glycerin diglycidyl ether.

The use amount of the internal crosslinking agent is preferably 30 mmol or less, more preferably 0.01 to 10 mmol, further more preferably 0.012 to 5 mmol, particularly preferably 0.015 to 1 mmol, extremely preferably 0.02 to 0.1 mmol, and extraordinarily preferably 0.025 to 0.06 mmol per 1 mol of the ethylenically unsaturated monomer, from the viewpoint of easily obtaining a better permeation rate in an absorbent article, and from the viewpoint of suppressing water-soluble property by appropriately crosslinking the obtained polymer to easily obtain the sufficient water absorption amount.

It is possible to perform heating while stirring in a state of mixing an ethylenically unsaturated monomer, a radical polymerization initiator, a surfactant, a polymeric dispersant, a hydrocarbon dispersion medium, or the like (if necessary, additionally an internal crosslinking agent), and to perform reverse phase suspension polymerization in a water-in-oil system.

When performing the reverse phase suspension polymerization, a monomer aqueous solution containing an ethylenically unsaturated monomer is dispersed in a hydrocarbon dispersion medium in the presence of a surfactant (if necessary, additionally a polymeric dispersant). At this time, before the start of the polymerization reaction, the timing of adding the surfactant, the polymeric dispersant, or the like may be either before or after the addition of the monomer aqueous solution.

Among these, from the viewpoint of easily reducing the amount of the hydrocarbon dispersion medium remaining in the obtained water-absorbent resin, it is preferable to perform polymerization after dispersing the monomer aqueous solution in the hydrocarbon dispersion medium in which the polymeric dispersant is dispersed and then further dispersing the surfactant.

Reverse phase suspension polymerization can be performed in one stage, or in multiple stages of two or more stages. Reverse phase suspension polymerization is preferably performed in two to three stages from the viewpoint of increasing productivity.

In a case where reverse phase suspension polymerization is performed in multiple stages of two or more stages, a first stage reverse phase suspension polymerization is performed, an ethylenically unsaturated monomer is added to the reaction mixture obtained in the first polymerization reaction and mixed therewith, and second and subsequent stages of reverse phase suspension polymerization may be performed in the same method as the first stage. In the reverse phase suspension polymerization in each stage of the second and subsequent stages, in addition to the ethylenically unsaturated monomer, the above-mentioned radical polymerization initiator and/or internal crosslinking agent is preferably added in a range of a molar ratio of each component with respect to the above-mentioned ethylenically unsaturated monomer, based on an amount of the ethylenically unsaturated monomer added at the time of the second and subsequent stages of reverse phase suspension polymerization, to perform reverse phase suspension polymerization. In the reverse phase suspension polymerization in each stage of second and subsequent stages, an internal crosslinking agent may be used if necessary. In a case of using the internal crosslinking agent, the internal crosslinking agent is preferably added within a range of the molar ratio of each component with respect to the above-mentioned ethylenically unsaturated monomer based on the amount of the ethylenically unsaturated monomer provided in each stage, to perform reverse phase suspension polymerization.

The temperature of the polymerization reaction varies depending on the used radical polymerization initiator, and the temperature is preferably 20°C to 150°C, and more preferably 40°C to 120°C, from the viewpoint of rapidly proceeding the polymerization and shortening the polymerization time to enhance economic efficiency, and easily removing polymerization heat and smoothly performing reaction. The reaction time is usually 0.5 to 4 hours. The completion of the polymerization reaction can be confirmed by stopping the temperature rise in the reaction system. Thus, the polymer of the ethylenically unsaturated monomer is usually obtained in a state of a hydrogel.

After the polymerization, a post-polymerization crosslinking agent may be added to the obtained hydrogel-like polymer and heated to perform crosslinking. By performing post-polymerization crosslinking, a degree of crosslinking of the hydrogel-like polymer can be increased, and water-absorbent characteristics (such as a water retention amount) can be further improved.

Examples of the post-polymerization crosslinking agent include polyols such as ethylene glycol, propylene glycol, 1,4-butanediol, trimethylolpropane, glycerin, polyoxyethylene glycol, polyoxypropylene glycol, and polyglycerin;

compounds having two or more epoxy groups, such as (poly)ethylene glycol diglycidyl ether, (poly)propylene glycol diglycidyl ether, and (poly)glycerin diglycidyl ether; haloepoxy compounds such as epichlorohydrin, epibromohydrin, and $\alpha$-methyl epichlorohydrin; compounds having two or more isocyanate groups such as 2,4-tolylene diisocyanate and hexamethylene diisocyanate; oxazoline compounds such as 1,2-ethylenebisoxazoline; carbonate compounds such as ethylene carbonate; and hydroxyalkylamide compounds such as bis[N,N-di($\beta$-hydroxyethyl)]adipamide. Among these, polyglycidyl compounds such as (poly)ethylene glycol diglycidyl ether, (poly)glycerin diglycidyl ether, (poly)glycerin triglycidyl ether, (poly)propylene glycol polyglycidyl ether, and polyglycerol polyglycidyl ether are preferable. The crosslinking agent may be used alone, or may be used in combination of two or more kinds thereof.

The amount of the post-polymerization crosslinking agent is preferably 30 mmol or less, more preferably 10 mmol or less, further more preferably 0.01 to 5 mmol, particularly preferably 0.012 to 1 mmol, extremely preferably 0.015 to 0.1 mmol, and extraordinarily preferably 0.02 to 0.05 mmol per 1 mol of the ethylenically unsaturated monomer, from the viewpoint of easily obtaining suitable water-absorbent characteristics (such as a water retention amount) by appropriately crosslinking the obtained hydrogel-like polymer.

The timing of adding the post-polymerization crosslinking agent may be after the polymerization of the ethylenically unsaturated monomer used for the polymerization, and in the case of multiple-stage polymerization, it is preferable to add after the multiple-stage polymerization. Considering fluctuation in water due to heat generation at the time of polymerization and after polymerization, retention due to process delay, opening of the system at the time of addition of the crosslinking agent, addition of water due to the addition of the crosslinking agent, or the like, the post-polymerization crosslinking agent is preferably added in a region of [water content (immediately after polymerization) $\pm$ 3% by mass] from the viewpoint of water content (to be described later).

Subsequently, the polymer particles (for example, polymer particles having a structural unit derived from an ethylenically unsaturated monomer) are obtained by drying in order to remove water from the obtained hydrogel-like polymer. Examples of a drying method include (a) a method of removing water by performing azeotropic distillation by heating from outside in a state where a hydrogel-like polymer is dispersed in a hydrocarbon dispersion medium, and refluxing the hydrocarbon dispersion medium, (b) a method of taking out a hydrogel-like polymer by decantation and drying under reduced pressure, and (c) a method of filtering the hydrogel-like polymer with a filter and drying under reduced pressure. Among these, it is preferable to use the method (a) due to the simplicity in the production process.

It is possible to adjust the particle diameter of water-absorbent resin particles by adjusting a rotation speed of a stirrer during the polymerization reaction, or by adding a flocculant into the system after the polymerization reaction or in the initial stage of drying. By adding a flocculant, it is possible to increase the particle diameter of the obtained water-absorbent resin particles. As the flocculant, an inorganic flocculant can be used. Examples of the inorganic flocculant (for example, powdered inorganic flocculant) include silica, zeolite, bentonite, aluminum oxide, talc, titanium dioxide, kaolin, clay, and hydrotalcite. From the viewpoint of better flocculation effect, the flocculant is preferably at least one selected from the group consisting of silica, aluminum oxide, talc, and kaolin.

In the reverse phase suspension polymerization, a method of adding the flocculant is preferably a method of preliminarily dispersing a flocculant in a hydrocarbon dispersion medium or water of the same type as that used in the polymerization, and then mixing into a hydrocarbon dispersion medium containing a hydrogel-like polymer under stirring.

The addition amount of the flocculant is preferably 0.001 to 1 part by mass, more preferably 0.005 to 0.5 part by mass, and further more preferably 0.01 to 0.2 parts by mass with respect to 100 parts by mass of the ethylenically unsaturated monomer used for the polymerization. In a case where the addition amount of the flocculant is within the above-mentioned range, water-absorbent resin particles having a target particle size distribution can be easily obtained.

In the production of the water-absorbent resin particles, it is preferable to perform surface crosslinking of a surface portion (surface and in the vicinity of surface) of a hydrogel-like polymer using a surface crosslinking agent in a drying step (water removing step) or any subsequent steps. By performing surface crosslinking, a gel flow rate, water-absorbent characteristics (such as a water retention amount) of the water-absorbent resin particles and the like are easily controlled. The surface crosslinking is preferably performed at the timing when the hydrogel-like polymer has a specific water content. The timing of surface crosslinking is preferably when the water content of the hydrogel-like polymer is 5% to 50% by mass, more preferably when the water content of the hydrogel-like polymer is 10% to 40% by mass, and further more preferably when the water content of the hydrogel-like polymer is 15% to 35% by mass. The water content (mass%) of the hydrogel-like polymer is calculated by the following formula.

$$\text{Water content} = [\text{Ww}/(\text{Ww} + \text{Ws})] \times 100$$

Ww: Water amount of a hydrogel-like polymer obtained by adding water amount used if necessary when mixing a flocculant, a surface crosslinking agent, or the like to an amount obtained by subtracting water amount discharged to the outside of the system in the drying step, from water amount contained in a monomer aqueous solution before polymerization in the entire polymerization step.

Ws: Solid content calculated from the charged amount of materials such as ethylenically unsaturated monomer, crosslinking agent, and initiator that constitute a hydrogel-like polymer.

Examples of the surface crosslinking agent include compounds having two or more reactive functional groups. Examples of the surface crosslinking agent include polyols such as ethylene glycol, propylene glycol, 1,4-butanediol, trimethylolpropane, glycerin, polyoxyethylene glycol, polyoxypropylene glycol, and polyglycerin; polyglycidyl compounds such as (poly)ethylene glycol diglycidyl ether, (poly)glycerin diglycidyl ether, (poly)glycerin triglycidyl ether, trimethylolpropane triglycidyl ether, (poly)propylene glycol polyglycidyl ether, and (poly)glycerol polyglycidyl ether; haloepoxy compounds such as epichlorohydrin, epibromohydrin, and $\alpha$-methyl epichlorohydrin; isocyanate compounds such as 2,4-tolylene diisocyanate and hexamethylene diisocyanate; oxetane compounds such as 3-methyl-3-oxetane methanol, 3-ethyl-3-oxetane methanol, 3-butyl-3-oxetane methanol, 3-methyl-3-oxetane ethanol, 3-ethyl-3-oxetane ethanol, and 3-butyl-3-oxetane ethanol; oxazoline compounds such as 1,2-ethylenebisoxazoline; carbonate compounds such as ethylene carbonate; and hydroxyalkylamide compounds such as bis[N,N-di($\beta$-hydroxyethyl)]adipamide. The surface crosslinking agent may be used alone, or may be used in combination of two or more kinds thereof. The surface crosslinking agent is preferably a polyglycidyl compound, and more preferably at least one selected from the group consisting of (poly)ethylene glycol diglycidyl ether, (poly)glycerin diglycidyl ether, (poly)glycerin triglycidyl ether, (poly)propylene glycol polyglycidyl ether, and polyglycerol polyglycidyl ether.

The use amount of the surface crosslinking agent is preferably 0.01 to 20 mmol, more preferably 0.05 to 10 mmol, further more preferably 0.1 to 5 mmol, particularly preferably 0.15 to 1 mmol, and extremely preferably 0.2 to 0.5 mmol per 1 mol of the ethylenically unsaturated monomer used for polymerization, from the viewpoint of easily obtaining suitable water-absorbent characteristics (such as a water retention amount).

After the surface crosslinking, it is possible to obtain polymer particles which are surface-crosslinked dried products by distilling off water and a hydrocarbon dispersion medium, drying under heating and reduced pressure, or the like with a known method.

The polymerization reaction can be carried out using various stirrers having a stirring blade. As the stirring blade, it is possible to use a flat plate blade, a lattice blade, a paddle blade, a propeller blade, an anchor blade, a turbine blade, a Pfaudler blade, a ribbon blade, a full zone blade, a max blend blade, or the like. A flat plate blade has a shaft (stirring shaft) and a flat plate portion (stirring portion) disposed around the shaft. The flat plate portion may have a slit or the like. In a case where the flat plate blade is used as the stirring blade, it is easy to uniformly carry out the crosslinking reaction in polymer particles, and it is easy to increase a gel flow rate and adjust it within a suitable range while maintaining water-absorbent characteristics such as a water retention amount.

In addition to the polymer particles, the water-absorbent resin particles of the present embodiment can further contain additional components such as a gel stabilizer, a metal chelating agent (ethylenediaminetetraacetic acid and a salt thereof, diethylenetriamine pentaacetate and a salt thereof, and the like, for example, diethylenetriamine pentasodium pentaacetate), and a flowability improver (lubricant) of polymer particles. Additional components can be disposed inside the polymer particles, on the surface of the polymer particles, or both thereof.

The water-absorbent resin particles may contain a plurality of inorganic particles disposed on the surface of the polymer particles. For example, by mixing the polymer particles and the inorganic particles, it is possible to dispose the inorganic particles on the surface of the polymer particles. The inorganic particles may be silica particles such as amorphous silica.

In a case where the water-absorbent resin particles include inorganic particles disposed on the surface of the polymer particles, the content of the inorganic particles may be within the following range based on the total mass of the polymer particles. The content of the inorganic particles may be 0.05% by mass or more, 0.1% by mass or more, 0.15% by mass or more, or 0.2% by mass or more. The content of the inorganic particles may be 5.0% by mass or less, 3.0% by mass or less, 1.0% by mass or less, or 0.5% by mass or less.

The inorganic particles here usually have a minute size as compared with the size of the polymer particles. For example, the average particle diameter of the inorganic particles may be 0.1 to 50 $\mu$m, 0.5 to 30 $\mu$m, or 1 to 20 $\mu$m. The average particle diameter can be measured by a pore electric resistance method or a laser diffraction/scattering method depending on the characteristics of the particles.

The absorber of the present embodiment contains the water-absorbent resin particles of the present embodiment. The absorber of the present embodiment may contain a fibrous substance, for example, is a mixture containing water-absorbent resin particles and the fibrous substance. For example, the structure of the absorber may be a structure in which the water-absorbent resin particles and the fibrous substance are uniformly mixed, may be a structure in which the water-absorbent resin particles are sandwiched between the fibrous substances formed in the form of a sheet or a layer, or may be other structures.

Examples of the fibrous substance include finely pulverized wood pulp; cotton; cotton linter; rayon; cellulosic fibers such as cellulose acetate; synthetic fibers such as polyamide, polyester and polyolefin; and a mixture of these fibers. The fibrous substance may be used alone, or may be used in combination of two or more. As the fibrous substance, hydrophilic fibers can be used.

In order to enhance the morphological retention before and during use of the absorber, the fibers may be adhered to each other by adding an adhesive binder to the fibrous substance. Examples of the adhesive binder include thermal bonding synthetic fibers, hot melt adhesives, and adhesive emulsions. The adhesive binder may be used alone, or may be used in combination of two or more.

Examples of the thermal bonding synthetic fiber include a total fusion type binder such as polyethylene, polypropylene, and an ethylene-propylene copolymer; and a non-total fusion type binder made of a side-by-side or core-sheath structure of polypropylene and polyethylene. In the above-mentioned non-total fusion type binder, only the polyethylene portion can be thermal-bonded.

Examples of the hot melt adhesive include a mixture of a base polymer such as ethylene-vinyl acetate copolymer, styrene-isoprene-styrene block copolymer, styrene-butadiene-styrene block copolymer, styrene-ethylene-butylene-styrene block copolymer, styrene-ethylene-propylene-styrene block copolymer, and amorphous polypropylene with a tackifier, a plasticizer, an antioxidant, or the like.

Examples of the adhesive emulsion include a polymerization product of at least one monomer selected from the group consisting of methyl methacrylate, styrene, acrylonitrile, 2-ethylhexyl acrylate, butyl acrylate, butadiene, ethylene, and vinyl acetate.

The absorber of the present embodiment may contain an inorganic powder (for example, amorphous silica), a deodorant, an antibacterial agent, a dye, a pigment, a fragrance, a sticking agent, or the like. In a case where the water-absorbent resin particles contain inorganic particles, the absorber may contain an inorganic powder in addition to the inorganic particles of the water-absorbent resin particles.

The shape of the absorber of the present embodiment may be a sheet shape, for example. The thickness of the absorber (for example, thickness of the sheet shaped absorber) may be 0.1 to 20 mm or 0.3 to 15 mm.

The content of the water-absorbent resin particles in the absorber is preferably within the following range based on a total mass of the absorber or a total of the water-absorbent resin particles and the fibrous substance. The content of the water-absorbent resin particles is preferably 2% by mass or more, 10% by mass or more, 20% by mass or more, or 50% by mass or more, from the viewpoint of easily obtaining sufficient water-absorbent characteristics. The content of the water-absorbent resin particles is 100% by mass or less, and from the viewpoint of easily obtaining sufficient water-absorbent characteristics, the content is preferably 80% by mass or less, 70% by mass or less, or 60% by mass or less. From these viewpoints, the content of the water-absorbent resin particles is preferably 2% to 100% by mass, 10% to 80% by mass, 20% to 70% by mass, or 50% to 60% by mass.

The content of the water-absorbent resin particles in the absorber is preferably 100 to 1000 g, more preferably 150 to 800 g, and further more preferably 200 to 700 g per 1 m$^2$ of the absorber from the viewpoint of easily obtaining sufficient water-absorbent characteristics. The content of the fibrous substance in the absorber is preferably 50 to 800 g, more preferably 100 to 600 g, and further more preferably 150 to 500 g per 1 m$^2$ of the absorber from the viewpoint of easily obtaining sufficient water-absorbent characteristics.

The absorbent article of the present embodiment includes an absorber of the present embodiment. Examples of other constituent members of the absorbent article of the present embodiment include a core wrap that retains an absorber and prevents falloff or flow of a constituent member of the absorber; a liquid permeable sheet disposed on the outermost part at the side where the liquid to be absorbed enters; and a liquid impermeable sheet disposed on the outermost part at the opposite side to the side where the liquid to be absorbed enters. Examples of the absorbent article include diapers (for example, paper diapers), toilet training pants, incontinence pads, sanitary materials (sanitary napkins, tampons, and the like), sweat pads, pet sheets, portal toilet members, and animal excrement treatment materials.

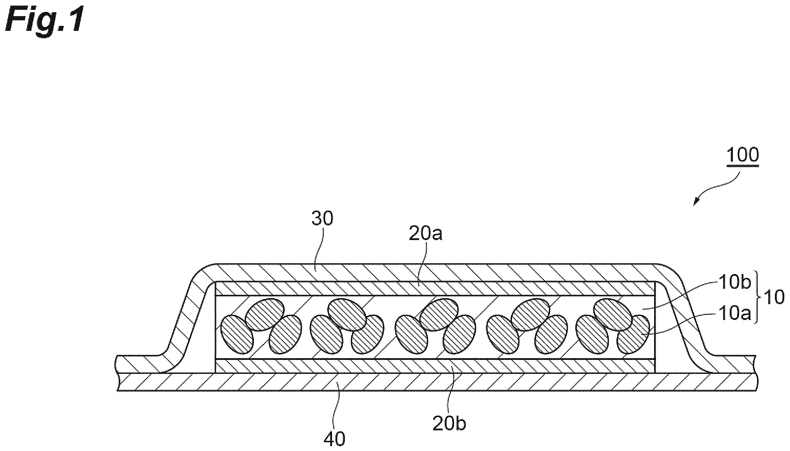

Fig. 1 is a cross-sectional view showing an example of an absorbent article. An absorbent article 100 shown in Fig. 1 includes an absorber 10, core wraps 20a and 20b, a liquid permeable sheet 30, and a liquid impermeable sheet 40. In the absorbent article 100, the liquid impermeable sheet 40, the core wrap 20b, the absorber 10, the core wrap 20a, and the liquid permeable sheet 30 are laminated in this order. In Fig. 1, there is a portion shown so that there is a gap between the members, but the members may be in close contact with each other without the gap.

The absorber 10 has a water-absorbent resin particle 10a of the present embodiment and a fiber layer 10b containing a fibrous substance. The water-absorbent resin particles 10a are dispersed in the fiber layer 10b.

The core wrap 20a is disposed on one surface side of the absorber 10 (an upper side of the absorber 10 in Fig. 1) in a state of being in contact with the absorber 10. The core wrap 20b is disposed on the other surface side of the absorber 10 (a lower side of the absorber 10 in Fig. 1) in a state of being in contact with the absorber 10. The absorber 10 is disposed between the core wrap 20a and the core wrap 20b. Examples of the core wraps 20a and 20b include tissues, non-woven fabrics, woven fabrics, synthetic resin films having liquid permeation holes, and net-like sheets having a mesh. The core wrap 20a and the core wrap 20b each have a main surface having the same size as that of the absorber 10, for example.

The liquid permeable sheet 30 is disposed on the outermost part at the side where the liquid to be absorbed enters. The liquid permeable sheet 30 is disposed on the core wrap 20a in a state of being in contact with the core wrap 20a. Examples of the liquid permeable sheet 30 include a non-woven fabric made of a synthetic resin such as polyethylene, polypropylene, polyester, and polyamide, and a porous sheet. The liquid impermeable sheet 40 is disposed on the outermost part at the opposite side to the liquid permeable sheet 30 in the absorbent article 100. The liquid impermeable sheet 40 is disposed on a lower side of the core wrap 20b in a state of being in contact with the core wrap 20b. Examples of the liquid impermeable sheet 40 include a sheet made of a synthetic resin such as polyethylene, polypropylene, and polyvinyl chloride, and a sheet made of a composite material of these synthetic resins and a non-woven fabric. The liquid permeable sheet 30 and the liquid impermeable sheet 40 have a main surface wider than the main surface of the absorber 10, and outer edges of the liquid permeable sheet 30 and the liquid impermeable sheet 40 are present around the absorber 10 and the core wraps 20a and 20b.

The magnitude relationship between the absorber 10, the core wraps 20a and 20b, the liquid permeable sheet 30, and the liquid impermeable sheet 40 is not particularly limited, and is appropriately adjusted according to the use of the absorbent article or the like. In addition, the method of retaining the shape of the absorber 10 using the core wraps 20a and 20b is not particularly limited, and as shown in Fig. 1, the absorber may be wrapped by a plurality of core wraps, and the absorber is wrapped by one core wrap.

The absorber may be adhered to a top sheet. In a case where the absorber is sandwiched or covered by the core wrap, it is preferable that at least the core wrap and the top sheet are adhered to each other, and it is more preferable that the core wrap and the top sheet are adhered to each other and the core wrap and the absorber are adhered to each other. Examples of a method of adhering the absorber include a method of adhering by applying a hot melt adhesive to the top sheet at predetermined intervals in a striped shape, a spiral shape, or the like in a width direction; and a method of adhering using a water-soluble binder such as starch, carboxymethyl cellulose, polyvinyl alcohol, polyvinylpyrrolidone, and other water-soluble polymers. In addition, in a case where the absorber contains thermal bonding synthetic fibers, a method of adhering by thermal bonding of the thermal bonding synthetic fibers may be adopted.

According to the present embodiment, it is possible to provide a liquid absorbing method using the water-absorbent resin particles, the absorber or the absorbent article of the present embodiment. The liquid absorbing method of the present embodiment includes a step of bringing the liquid to be absorbed into contact with the water-absorbent resin particles, the absorber or the absorbent article of the present embodiment.

According to the present embodiment, it is possible to provide a method for adjusting a permeation rate (a permeation rate of a liquid) in an absorbent article, which is a method for adjusting (for example, a method of increasing) a permeation rate using the water-absorbent resin particles, the absorber, or the absorbent article of the present embodiment. The method for adjusting a permeation rate of the present embodiment includes an adjustment step of adjusting a gel flow rate measured by the above-mentioned procedures having (1) to (3) relating to the water-absorbent resin particles of the present embodiment. In the adjustment step, the gel flow rate can be adjusted within each of the above-mentioned ranges (for example, 10 to 70 g/min).

According to the present embodiment, it is possible to provide a method for producing water-absorbent resin particles, the method including a selection step of selecting water-absorbent resin particles based on a gel flow rate measured by the above-mentioned procedures having (1) to (3) relating to the water-absorbent resin particles of the present embodiment. In the selection step, the gel flow rate can be adjusted within each of the above-mentioned ranges (for example, 10 to 70 g/min).

According to the present embodiment, it is possible to provide a method for producing an absorber by using the water-absorbent resin particles obtained by the above-mentioned method for producing water-absorbent resin particles. The method for producing an absorber of the present embodiment includes a particle producing step of obtaining water-absorbent resin particles by the above-mentioned method for producing water-absorbent resin particles. The method for producing an absorber of the present embodiment may include a step of mixing the water-absorbent resin particles and a fibrous substance after the particle producing step. According to the present embodiment, it is possible to provide a method for producing an absorbent article by using the absorber obtained by the above-mentioned method for producing an absorber. The method for producing an absorbent article of the present embodiment includes an absorber producing step of obtaining an absorber by the above-mentioned method for producing an absorber. The method for producing an absorbent article of the present embodiment may include a step of obtaining an absorbent article by using the absorber and other constituent member for an absorbent article after the absorber producing step, and in this step, for example, an absorbent article is obtained by laminating the absorber and other constituent member for an absorbent article with each other.

## Examples

Hereinafter, contents of the present invention will be described in further detail using examples and comparative examples, but the present invention is not limited to the following examples.

<Production of water-absorbent resin particles>

(Example 1)

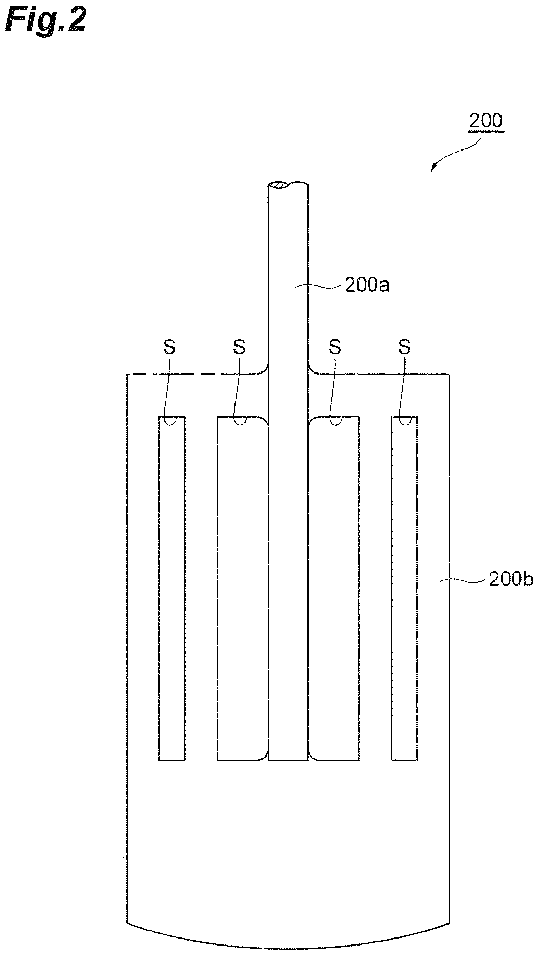

A round-bottomed cylindrical separable flask with the inner diameter of 11 cm and the internal volume of 2 L equipped with a reflux cooling device, a dropping funnel, a nitrogen gas introduction tube, and a stirrer was prepared. The stirrer was equipped with a stirring blade (flat plate blade) 200 of which an outline is shown in Fig. 2. The stirring blade 200 includes a shaft 200a and a flat plate portion 200b. The flat plate portion 200b is welded to the shaft 200a and has a curved tip end. Four slits S extending along an axial direction of the shaft 200a are formed in the flat plate portion 200b. The four slits S are arranged in a width direction of the flat plate portion 200b, where the width of the two slits S at the inner side is 1 cm, and the width of the two slits S at the outer side is 0.5 cm. The length of the flat plate portion 200b is about 10 cm, and the width of the flat plate portion 200b is about 6 cm. Subsequently, into the above-mentioned separable flask, 293 g of n-heptane was added as a hydrocarbon dispersion medium, and 0.736 g of a maleic anhydride-modified ethylene-propylene copolymer (manufactured by Mitsui Chemicals, Inc., High Wax 1105A) was added as a polymeric dispersant to obtain a mixture. The dispersant was dissolved by raising the temperature to 80°C while stirring the mixture, and then the mixture was cooled to 50°C.

Subsequently, 92.0 g of an aqueous solution of 80.5% by mass acrylic acid (acrylic acid: 1.03 mol) was added into a beaker having the internal volume of 300 mL as a water-soluble ethylenically unsaturated monomer. Subsequently, while cooling from the outside, 147.7 g of an aqueous solution of 20.9% by mass sodium hydroxide was added dropwise into the beaker to perform 75 mol% of neutralization. Thereafter, 0.092 g of hydroxyethyl cellulose (manufactured by Sumitomo Seika Chemicals Co., Ltd., HEC AW-15F) as a thickener, 0.092 g (0.339 mmol) of 2,2'-azobis(2-amidinopropane) dihydrochloride and 0.018 g (0.067 mmol) of potassium persulfate as a water-soluble radical polymerization initiator, and 0.0046 g (0.026 mmol) of ethylene glycol diglycidyl ether as an internal crosslinking agent were added and dissolved to prepare a first stage aqueous liquid.

Then, the above-mentioned first stage aqueous liquid was added into the above-mentioned separable flask while stirring at the rotation speed of 425 rpm of the stirrer, and then stirring was performed for 10 minutes. Thereafter, a surfactant solution obtained by heat-dissolving 0.736 g of sucrose stearic acid ester (surfactant, manufactured by Mitsubishi-Chemical Foods Corporation, Ryoto Sugar Ester S-370, HLB value: 3) in 6.62 g of n-heptane was added into the separable flask. Then, the inside of the system was sufficiently replaced with nitrogen while stirring at the rotation speed of 425 rpm of the stirrer. Thereafter, the flask was immersed in a water bath at 70°C to raise the temperature, and polymerization was performed for 60 minutes to obtain a first stage polymerization slurry solution.

Subsequently, 128.8 g of an aqueous solution of 80.5% by mass acrylic acid (acrylic acid: 1.43 mol) was added into another beaker having the internal volume of 500 mL as a water-soluble ethylenically unsaturated monomer. Subsequently, while cooling from the outside, 159.0 g of an aqueous solution of 27% by mass sodium hydroxide was added dropwise into the beaker to perform 75 mol% of neutralization. Thereafter, 0.129 g (0.476 mmol) of 2,2'-azobis(2-amidinopropane) dihydrochloride and 0.026 g (0.096 mmol) of potassium persulfate as a water-soluble radical polymerization initiator, and 0.0117 g (0.067 mmol) of ethylene glycol diglycidyl ether as an internal crosslinking agent were added and then dissolved to prepare a second stage aqueous liquid.

Subsequently, while stirring at the rotation speed of 650 rpm of the stirrer, the inside of the above-mentioned separable flask was cooled to 25°C, and then the total amount of the above-mentioned second stage aqueous liquid was added to the above-mentioned first stage polymerization slurry solution. Subsequently, after replacing the inside of the system with nitrogen for 30 minutes, the flask was immersed in a water bath at 70°C again to raise the temperature, and the polymerization reaction was performed for 60 minutes to obtain a second stage hydrogel-like polymer.

To the above-mentioned second stage hydrogel-like polymer, 0.589 g of an aqueous solution of 45% by mass diethylenetriamine pentaacetic acid pentasodium was added under stirring. Thereafter, the temperature of the reaction solution was raised in an oil bath at 125°C, and 207.9 g of water was extracted to the outside of the system while refluxing n-heptane by azeotropic distillation of n-heptane and water. Then, 4.42 g of an aqueous solution of 2% by mass ethylene glycol diglycidyl ether (ethylene glycol diglycidyl ether: 0.507 mmol) was added into the flask as a surface crosslinking agent, and then the mixture was maintained at 83°C for 2 hours.

Thereafter, drying was performed by evaporating n-heptane at 125°C to obtain polymer particles (dried product). After passing these polymer particles through a sieve having the opening of 850 $\mu$m, 0.2% by mass of amorphous silica (Tokusil NP-S manufactured by Oriental Silicas Corporation) was mixed with the polymer particles based on the total mass of the polymer particles to obtain 232.3 g of water-absorbent resin particles containing amorphous silica. The median particle diameter of the water-absorbent resin particles was 361 $\mu$m.

(Example 2)

231.0 g of water-absorbent resin particles were obtained in the same manner as that in Example 1, except that, in the hydrogel-like polymer obtained after the second stage polymerization, 224.3 g of water was extracted to the outside of the system by azeotropic distillation. The median particle diameter of the water-absorbent resin particles was 342 μm.

(Example 3)

232.1 g of water-absorbent resin particles were obtained in the same manner as that in Example 1, except that, in the hydrogel-like polymer obtained after the second stage polymerization, 234.6 g of water was extracted to the outside of the system by azeotropic distillation. The median particle diameter of the water-absorbent resin particles was 355 μm.

(Comparative Example 1)

231.0 g of water-absorbent resin particles was obtained in the same manner as in Example 1 except that, when dissolving a maleic anhydride-modified ethylene-propylene copolymer, a stirring blade having two stages of four inclined paddle blades with the blade diameter of 5 cm was used as a stirrer instead of the flat plate blade; in preparation of a first stage aqueous liquid, 2,2'-azobis(2-amidinopropane) dihydrochloride was not used and 0.0736 g (0.272 mmol) of potassium persulfate was used as a water-soluble radical polymerization initiator, and 0.010 g (0.057 mmol) of ethylene glycol diglycidyl ether was used as an internal crosslinking agent; in preparation of a first stage polymerization slurry solution, the rotation speed of the stirrer was changed to 550 rpm; in preparation of a second stage aqueous liquid, 2,2'-azobis(2-amidinopropane) dihydrochloride was not used and 0.090 g (0.333 mmol) of potassium persulfate was used as a water-soluble radical polymerization initiator, and 0.0117 g (0.067 mmol) of ethylene glycol diglycidyl ether was used as an internal crosslinking agent; when adding the total amount of the second stage aqueous liquid to the first stage polymerization slurry solution, the rotation speed of the stirrer was changed to 1000 rpm; the use amount of an aqueous solution of 45% by mass diethylenetriaminepentaacetic acid pentasodium to be added to a hydrogel-like polymer obtained after the second stage polymerization was changed to 0.265 g, and 247.9 g of water was extracted out of the system by azeotropic distillation; and 0.5% by mass of amorphous silica with respect to a mass of polymer particles was mixed with the polymer particles. The median particle diameter of the water-absorbent resin particles was 355 μm.

(Comparative Example 2)

230.8 g of water-absorbent resin particles were obtained in the same manner as that in Comparative Example 1, except that, in the hydrogel-like polymer obtained after the second stage polymerization, 253.9 g of water was extracted to the outside of the system by azeotropic distillation, and 0.1% by mass amorphous silica with respect to the mass of the polymer particles was mixed with the polymer particles. The median particle diameter of the water-absorbent resin particles was 360 μm.

(Comparative Example 3)

230.6 g of water-absorbent resin particles was obtained in the same manner as in Comparative Example 1 except that, in a hydrogel-like polymer obtained after the second stage polymerization, 271.4 g of water was extracted to the outside of the system by azeotropic distillation, and the use amount of an aqueous solution of 2% by mass ethylene glycol diglycidyl ether to be added after the azeotropic distillation was changed to 6.40 g (0.735 mmol). The median particle diameter of the water-absorbent resin particles was 355 μm.

(Comparative Example 4)

230.8 g of water-absorbent resin particles were obtained in the same manner as that in Comparative Example 1, except that, in the hydrogel-like polymer obtain after the second stage polymerization, 278.9 g of water was extracted to the outside of the system by azeotropic distillation, and 0.2% by mass amorphous silica with respect to the mass of the polymer particles was mixed with the polymer particles. The median particle diameter of the water-absorbent resin particles was 358 μm.

(Comparative Example 5)

A round-bottomed cylindrical separable flask with the inner diameter of 11 cm and the internal volume of 2 L equipped with a reflux cooling device, a dropping funnel, a nitrogen gas introduction tube, and a stirrer (a stirring blade having two stages of four inclined paddle blades with the blade diameter of 5 cm) was prepared. Into this flask, 300 g of n-heptane as a hydrocarbon dispersion medium was added and 0.74 g of a maleic anhydride-modified ethylene-propylene copolymer (manufactured by Mitsui Chemicals, Inc., High Wax 1105A) as a polymeric dispersant was added to obtain a mixture. The dispersant was heated and dissolved while stirring this mixture, and then the mixture was cooled to 50°C.

Subsequently, 92 g of an aqueous solution of 80% by mass acrylic acid (acrylic acid: 1.02 mol) was added into an Erlenmeyer flask having the internal volume of 500 mL as a water-soluble ethylenically unsaturated monomer. Subsequently, while cooling from the outside, 102.2 g of an aqueous solution of 30% by mass sodium hydroxide was added dropwise into the flask to perform 75 mol% of neutralization. Thereafter, 0.092 g of hydroxyethyl cellulose (manufactured by Sumitomo Seika Chemicals Co., Ltd., HEC AW-15F) as a thickener, 0.110 g (0.407 mmol) of 2,2'-azobis(2-amidinopropane) dihydrochloride and 0.009 g (0.034 mmol) of potassium persulfate as a water-soluble radical polymerization initiator, 0.006 g (0.037 mmol) of ethylene glycol diglycidyl ether as an internal crosslinking agent, and 48.0 g of ion-exchanged water were added and dissolved to prepare a first stage aqueous liquid.

Then, the above-mentioned first stage aqueous liquid was added into the above-mentioned separable flask while stirring at the rotation speed of 550 rpm of the stirrer, and then stirring was performed for 10 minutes. Thereafter, 7.4 g of a surfactant solution obtained by heat-dissolving 0.74 g of sucrose stearic acid ester (surfactant, manufactured by Mitsubishi-Chemical Foods Corporation, Ryoto Sugar Ester S-370, HLB value: 3) in 6.66 g of n-heptane was added. Then, the inside of the system was sufficiently replaced with nitrogen while stirring at the rotation speed of 550 rpm of the stirrer. Thereafter, the flask was immersed in a water bath at 70°C to raise the temperature, and polymerization was performed for 60 minutes to obtain a first stage polymerization slurry solution.

Subsequently, 128.8 g of an aqueous solution of 80% by mass acrylic acid (acrylic acid: 1.43 mol) was added into another Erlenmeyer flask having the internal volume of 500 mL as a water-soluble ethylenically unsaturated monomer. Subsequently, while cooling from the outside, 143.1 g of an aqueous solution of 30% by mass sodium hydroxide was added dropwise into the Erlenmeyer flask to perform 75 mol% of neutralization. Thereafter, 0.155 g (0.570 mmol) of 2,2'-azobis(2-amidinopropane) dihydrochloride and 0.013 g (0.048 mmol) of potassium persulfate as a water-soluble radical polymerization initiator, 0.009 g (0.052 mmol) of ethylene glycol diglycidyl ether as an internal crosslinking agent, and 12.5 g of ion-exchanged water were added and then dissolved to prepare a second stage aqueous liquid.

Subsequently, while stirring at the rotation speed of 1000 rpm of the stirrer, the inside of the above-mentioned separable flask was cooled to 25°C, and then the total amount of the above-mentioned second stage aqueous liquid was added to the above-mentioned first stage polymerization slurry solution. Subsequently, after sufficiently replacing the inside of the system with nitrogen, the flask was immersed in a water bath at 70°C again to raise the temperature, and second stage polymerization was performed for 30 minutes.

After the second stage polymerization, the temperature of the second stage reaction solution was raised in an oil bath at 125°C, and 245 g of water was extracted to the outside of the system while refluxing n-heptane by azeotropic distillation of n-heptane and water. Then, drying was performed by evaporating n-heptane to obtain polymer particles (dried product). These polymer particles were passed through a sieve having the opening of 1000 $\mu$m to obtain 233.2 g of water-absorbent resin particles. The median particle diameter of the water-absorbent resin particles was 370 $\mu$m.

<Median particle diameter>

The above-mentioned median particle diameter of the water-absorbent resin particles was measured by the following procedure. That is, JIS standard sieves were combined in the following order from the top: a sieve having the opening of 600 $\mu$m, a sieve having the opening of 500 $\mu$m, a sieve having the opening of 425 $\mu$m, a sieve having the opening of 300 $\mu$m, a sieve having the opening of 250 $\mu$m, a sieve having the opening of 180 $\mu$m, a sieve having the opening of 150 $\mu$m, and a tray. 50 g of the water-absorbent resin particles was put in the topmost sieve among the combined sieves, and classification was performed using a Rotap shaker (manufactured by Iida-seisakusho Japan Corporation) according to JIS Z 8815 (1994). After the classification, a mass of the particles remaining on each of the sieves was calculated as a mass percentage with respect to the total amount to determine a particle size distribution. The relationship between the opening of the sieve and the integrated value of the mass percentage of the particles remaining on the sieve was plotted on a logarithmic probability paper by integrating in the order from the one having the largest particle diameter on the sieve with respect to this particle size distribution. By connecting the plots on the probability paper with a straight line, the particle diameter corresponding to the cumulative mass percentage of 50% by mass was obtained as the median particle diameter.

<Water retention amount of water-absorbent resin particles>

The water retention amount (at room temperature, 25°C ± 2°C) of physiological saline of the water-absorbent resin particles was measured by the following procedure. First, a cotton bag (Cotton broadcloth No. 60, 100 mm in width × 200 mm in length) into which 2.0 g of the water-absorbent resin particles had been weighed was placed in a beaker having the internal volume of 500 mL. After pouring 500 g of physiological saline into the cotton bag containing the water-absorbent resin particles at one time so that lumps could not be produced, the upper part of the cotton bag was bound with a rubber band and the cotton bag was left to stand for 30 minutes to swell the water-absorbent resin particles. The cotton bag after an elapse of 30 minutes was dehydrated for 1 minute using a dehydrator (manufactured by KOKUSAN Co., Ltd., product number: H-122) which had been set to have the centrifugal force of 167 G, and then the mass Wa [g] of the cotton bag containing the swollen gel after dehydration was measured. The same operation was performed without addition of the water-absorbent resin particles, the empty mass Wb [g] at the time when the cotton bag was wet was measured, and the water retention amount of physiological saline of the water-absorbent resin particles was calculated from the following formula. The results are shown in Table 1.

$$\text{Water retention amount } [g/g] = (Wa - Wb)/2.0$$

<Water absorption amount of water-absorbent resin particles under load>

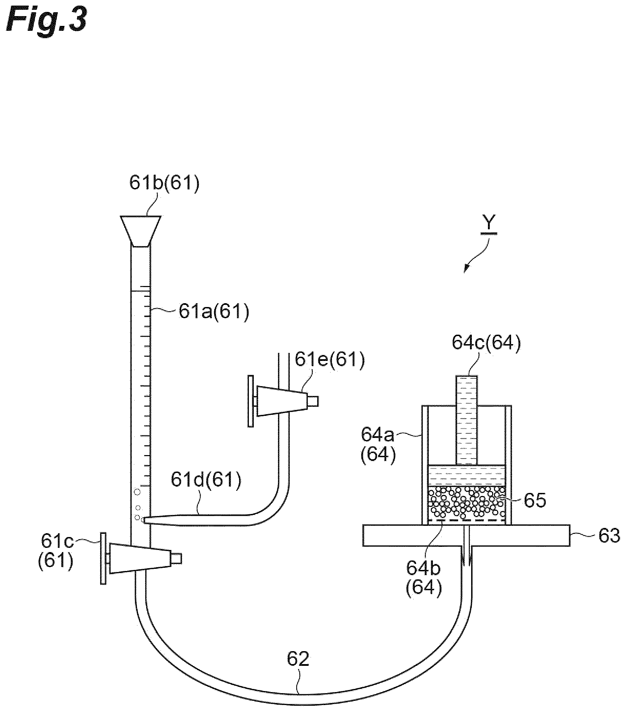

A water absorption amount of physiological saline of the water-absorbent resin particles under a load (at room temperature, 25°C $\pm$ 2°C) was measured using a measurement device Y shown in Fig. 3. The measurement device Y is constituted of a burette unit 61, a conduit 62, a measurement table 63, and a measurement unit 64 placed on the measurement table 63. The burette unit 61 has a burette 61a extending in a vertical direction, a rubber stopper 61b disposed at the upper end of the burette 61a, a cock 61c disposed at the lower end of the burette 61a, an air introduction tube 61d of which one end extends into the burette 61a in the vicinity of the cock 61c, and a cock 61e disposed on the other end side of the air introduction tube 61d. The conduit 62 is attached between the burette unit 61 and the measurement table 63. The inner diameter of the conduit 62 is 6 mm. At the central portion of the measurement table 63, a hole having the diameter of 2 mm is formed and the conduit 62 is connected. The measurement unit 64 has a cylinder 64a (made of acrylic resin), a nylon mesh 64b adhered to the bottom of the cylinder 64a, and a weight 64c. The inner diameter of the cylinder 64a is 20 mm. The opening of the nylon mesh 64b is 75 $\mu$m (200 mesh). Then, at the time of measurement, the water-absorbent resin particles 66 to be measured are uniformly scattered on the nylon mesh 64b. The weight 64c has the diameter of 19 mm and the mass of the weight 64c is 119.6 g. The weight 64c is placed on the water-absorbent resin particles 66, and can apply the load of 4.14 kPa to the water-absorbent resin particles 66.

After putting 0.100 g of the water-absorbent resin particles 66 in the cylinder 64a of the measurement device Y, the weight 64c was placed and the measurement was started. Since the same volume of air as physiological saline absorbed by the water-absorbent resin particles 66 is quickly and smoothly supplied to the inside of the burette 61a from the air introduction tube, the amount of reduction in the water level of physiological saline inside the burette 61a corresponds to the amount of physiological saline absorbed by the water-absorbent resin particles 66. A scale of the burette 61a is engraved from top to bottom in increments of 0 mL to 0.5 mL; as a water level of physiological saline, a scale Va of the burette 61a before the start of water absorption and a scale Vb of the burette 61a after 120 minutes from the start of water absorption are read; and a water absorption amount under the load was calculated by the following formula. The results are shown in Table 1.

$$\text{Water absorption amount under load } [mL/g] = (Vb - Va)/0.1$$

<Gel flow rate>

(Preparation of swollen gel)

398 g of ion-exchanged water was weighed into a beaker having the internal volume of 1000 mL. Next, while stirring the ion-exchanged water at 1000 rpm using a magnetic stirrer bar (8 mm$\varphi$ $\times$ 30 mm, without a ring), 2.0 g of the water-absorbent resin particles were dispersed in the ion-exchanged water (at room temperature (25°C $\pm$ 2°C)) so that lumps could not be produced. The mixture was left to stand for 30 minutes while continuing stirring to sufficiently swell the water-absorbent resin particles, and thereby a swollen gel was obtained.

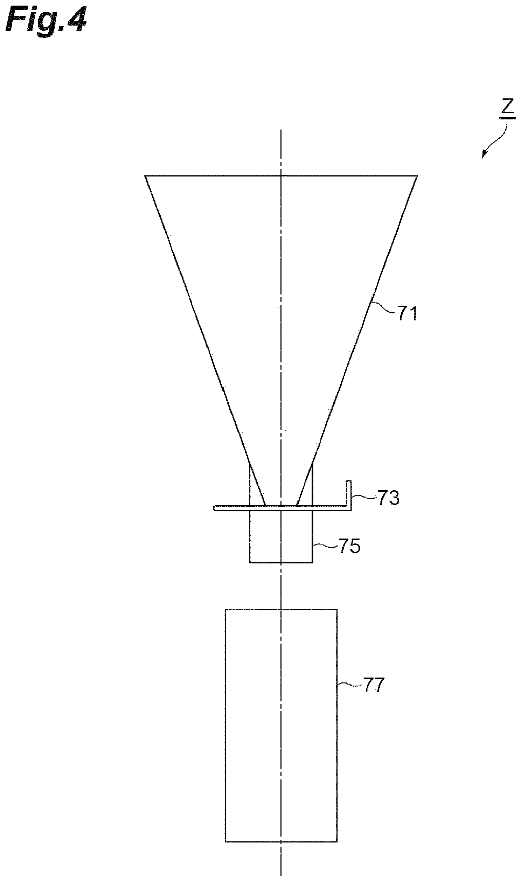

The gel flow rate (fluidity of the swollen gel, at room temperature (25°C $\pm$ 2°C)) was measured using a measurement device Z shown in Fig. 4. The measurement device Z includes a funnel 71, a damper 73, a cylinder 75, and a receiver 77.

The funnel 71 has, as an opening, a supply port (upper end opening, diameter (inner diameter): 91 mm) for supplying the swollen gel, and a discharge port (lower end opening, diameter (inner diameter): 8 mm) for discharging the swollen gel, and has a tapered shape that narrows from the supply port to the discharge port. The inclination angle of a side wall of the funnel 71 is 20° with respect to an axial direction of the funnel 71. The length from the supply port to the discharge port in the funnel 71 is 114 mm. The cross-section that is vertical to the axial direction of the funnel 71, including the supply port and the discharge port, is circular. The constituent material of the funnel 71 is stainless steel.

The funnel 71 is fixed by gripping an outer circumferential part of the supply port side with a support ring.

The damper 73 is a member having the length of 47 mm and the width of 15 mm. The damper 73 is disposed at the discharge port of the funnel 71, and opens and closes the discharge port of the funnel 71. The cylinder 75 is a member having the length of 35 mm and the inner diameter of 20 mm. The cylinder 75 is disposed on the discharge port side of the funnel 71 so that a portion on the discharge port side of the funnel 71 is located inside. The receiver 77 is a beaker (manufactured by SANPLATEC CO., LTD., Sanpla-cup, 500 mL) having the internal volume of 500 mL.

First, it was confirmed that an upper end surface of the funnel 71 of the measurement device Z was located horizontally with respect to a laboratory table using a level gauge. Next, in a state where the discharge port was closed by the damper 73, 250 g of the above-mentioned swollen gel was accommodated in the funnel 71, and then left to stand for 3 minutes. In a state where an upper end of the receiver 77 was located 38 mm below the damper 73 (discharge port), the discharge port was opened by quickly pulling out the damper 73. After a lapse of 3 minutes from the opening, the receiver 77 was slid to remove the receiver 77 from the location which was below the discharge port and from which the gel flowed out, and a mass (gel flow amount) of the swollen gel flowed out to the receiver 77 was measured. Then, an outflow amount per unit time was calculated as a gel flow rate from the following formula. The results are shown in Table 1.

$$\text{Gel flow rate [g/min] = gel flow amount [g]/3 [min]}$$

<Production of absorbent article>

10 g of the water-absorbent resin particles and 6.8 g of pulverized pulp were uniformly mixed by air papermaking using an air flow type mixer (Padformer manufactured by O-tec Co., Ltd.), and thereby a sheet-shaped absorber having the size of 40 cm × 12 cm was produced. Subsequently, in a state where an absorber in a sheet shape was sandwiched from its upper and lower sides with two sheets of tissue paper having the basis weight of 16 g/m$^2$ and having the same size as that of the absorber, a laminate was obtained by applying the load of 196 kPa to the entire absorber for 30 seconds to press. Furthermore, an airthrough type porous liquid permeable sheet, which was made of polyethylene-polypropylene, had the basis weight of 22 g/m$^2$, and had the same size as that of the absorber, was disposed on the upper surface of the laminate, and thereby an absorbent article was produced.

<Permeation rate>

The absorbent article was disposed on a horizontal table in a room at the temperature of 25°C ± 2°C. Next, a liquid injection cylinder (cylinder with both ends open) having the volume of 100 mL and having an inlet with the inner diameter of 3 cm was placed at the central part of a main surface of the absorbent article. Subsequently, 80 mL of physiological saline, which had been previously colored with a small amount of Blue No. 1 and adjusted to 25°C ± 1°C, was injected into the cylinder at one time (supplied from the vertical direction). Using a stopwatch, an absorption time from the start of the injection until the physiological saline completely disappeared from the cylinder was measured. This operation was performed twice more at intervals of 30 minutes (three times in total), and an absorption time of the third time was obtained as a permeation rate [seconds]. A permeation rate is more preferable as it becomes shorter. The results are shown in Table 1.

[Table 1]

| | Water-absorbent resin particles | | | Absorbent article |
|---|---|---|---|---|
| | Water retention amount [g/g] | Water absorption amount under load [mL/g] | Gel flow rate [g/min] | Permeation rate |
| Example 1 | 34 | 28 | 46.5 | 80 |
| Example 2 | 44 | 21 | 45.5 | 90 |
| Example 3 | 51 | 18 | 50.6 | 90 |
| Comparative Example 1 | 35 | 25 | 0.3 | 115 |
| Comparative Example 2 | 41 | 23 | 0.4 | 134 |

(continued)

| | Water-absorbent resin particles | | | Absorbent article |
|---|---|---|---|---|
| | Water retention amount [g/g] | Water absorption amount under load [mL/g] | Gel flow rate [g/min] | Permeation rate |
| Comparative Example 3 | 45 | 11 | 3.9 | 161 |
| Comparative Example 4 | 50 | 11 | 2.4 | 203 |
| Comparative Example 5 | 66 | 2 | 83.0 | 380 |

According to Table 1, it was confirmed that adjusting a gel flow rate is effective in obtaining an absorbent article having a better permeation rate.

### Reference Signs List

10: absorber, 10a, 66: water-absorbent resin particle, 10b: fiber layer, 20a, 20b: core wrap, 30: liquid permeable sheet, 40: liquid impermeable sheet, 61: burette unit, 61a: burette, 61b: rubber stopper, 61c: cock, 61d: air introduction tube, 61e: cock, 62: conduit, 63: measurement table, 64: measurement unit, 64a: cylinder, 64b: nylon mesh, 64c: weight, 71: funnel, 73: damper, 75: cylinder, 77: receiver, 100: absorbent article, 200: stirring blade, 200a: shaft, 200b: flat plate portion, S: slit, Y, Z: measurement device.

### Claims

1. Water-absorbent resin particles,
   wherein a gel flow rate measured by the following procedures having (1) to (3) is 10 to 70 g/min,

   (1) mixing 1 part by mass of the water-absorbent resin particles and 199 parts by mass of water to obtain a gel,
   (2) accommodating the gel in a funnel in a state where a circular lower end opening with a diameter of 8 mm in the funnel is closed, and
   (3) measuring an outflow amount of the gel per unit time after a lapse of 3 minutes from opening of the lower end opening as the gel flow rate.

2. The water-absorbent resin particles according to claim 1, wherein a water absorption amount of physiological saline under a load of 4.14 kPa is 10 to 40 mL/g.

3. The water-absorbent resin particles according to claim 1 or 2, wherein the water-absorbent resin particles comprise a structural unit derived from at least one selected from the group consisting of (meth)acrylic acid and a salt thereof.

4. An absorber comprising the water-absorbent resin particles according to any one of claims 1 to 3.

5. The absorber according to claim 4, wherein a content of the water-absorbent resin particles is 50% by mass or more based on a total mass of the absorber.

6. An absorbent article comprising the absorber according to claim 4 or 5.

7. The absorbent article according to claim 6, which is a diaper.

8. A method for producing water-absorbent resin particles, the method comprising
   selecting water-absorbent resin particles based on a gel flow rate measured by the following procedures having (1) to (3),

   (1) mixing 1 part by mass of the water-absorbent resin particles and 199 parts by mass of water to obtain a gel,

(2) accommodating the gel in a funnel in a state where a circular lower end opening with a diameter of 8 mm in the funnel is closed, and

(3) measuring an outflow amount of the gel per unit time after a lapse of 3 minutes from opening of the lower end opening as the gel flow rate.

9. A method for adjusting a permeation rate in an absorbent article comprising an absorber,

wherein the absorber comprises water-absorbent resin particles, and

the method comprises adjusting a gel flow rate measured by the following procedures having (1) to (3),

(1) mixing 1 part by mass of the water-absorbent resin particles and 199 parts by mass of water to obtain a gel,

(2) accommodating the gel in a funnel in a state where a circular lower end opening with a diameter of 8 mm in the funnel is closed, and

(3) measuring an outflow amount of the gel per unit time after a lapse of 3 minutes from opening of the lower end opening as the gel flow rate.

Fig.1

# *Fig.2*

*Fig.3*

# Fig.4

## INTERNATIONAL SEARCH REPORT

| International application No. |
| --- |
| PCT/JP2020/009508 |

### A. CLASSIFICATION OF SUBJECT MATTER

C08F 20/06(2006.01)i; C08J 3/24(2006.01)i; A61F 13/53(2006.01)i; A61F 13/84(2006.01)i; A61L 15/60(2006.01)i
FI: C08F20/06; A61F13/84 100; A61L15/60; C08J3/24 Z CEY; A61F13/53 300
According to International Patent Classification (IPC) or to both national classification and IPC

### B. FIELDS SEARCHED

Minimum documentation searched (classification system followed by classification symbols)
C08F20/06; C08J3/24; A61F13/53; A61F13/84; A61L15/60

| Documentation searched other than minimum documentation to the extent that such documents are included in the fields searched | |
| --- | --- |
| Published examined utility model applications of Japan | 1922–1996 |
| Published unexamined utility model applications of Japan | 1971–2020 |
| Registered utility model specifications of Japan | 1996–2020 |
| Published registered utility model applications of Japan | 1994–2020 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
| --- | --- | --- |
| X | JP 2006-176570 A (SUMITOMO SEIKA CHEMICALS CO., LTD.) 06.07.2006 (2006-07-06) examples | 1-7 |
| A | examples | 8-9 |
| X | WO 2006/123561 A1 (SUMITOMO SEIKA CHEMICALS CO., LTD.) 23.11.2006 (2006-11-23) examples | 1-7 |
| A | examples | 8-9 |
| X | WO 2012/144564 A1 (SUMITOMO SEIKA CHEMICALS CO., LTD.) 26.10.2012 (2012-10-26) examples | 1-7 |
| A | examples | 8-9 |
| X | WO 2016/006135 A1 (SUMITOMO SEIKA CHEMICALS CO., LTD.) 14.01.2016 (2016-01-14) examples | 1-7 |
| A | examples | 8-9 |

☒ Further documents are listed in the continuation of Box C.     ☒ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 19 May 2020 (19.05.2020) | 26 May 2020 (26.05.2020) |

| Name and mailing address of the ISA/ | Authorized officer |
| --- | --- |
| Japan Patent Office 3-4-3, Kasumigaseki, Chiyoda-ku, Tokyo 100-8915, Japan | |
| | Telephone No. |

Form PCT/ISA/210 (second sheet) (January 2015)

| International application No. |
| --- |
| PCT/JP2020/009508 |

C (Continuation).   DOCUMENTS CONSIDERED TO BE RELEVANT

| Category* | Citation of document, with indication, where appropriate, of the relevant passages | Relevant to claim No. |
| --- | --- | --- |
| X | JP 2016-28113 A (SUMITOMO SEIKA CHEMICALS CO., LTD.) 25.02.2016 (2016-02-25) examples | 1-7 |
| A | examples | 8-9 |
| X | JP 2016-28117 A (SUMITOMO SEIKA CHEMICALS CO., LTD.) 25.02.2016 (2016-02-25) examples | 1-7 |
| A | examples | 8-9 |

Form PCT/ISA/210 (continuation of second sheet) (January 2015)

**INTERNATIONAL SEARCH REPORT**
Information on patent family members

International application No.

PCT/JP2020/009508

| Patent Documents referred in the Report | Publication Date | Patent Family | Publication Date |
|---|---|---|---|
| JP 2006-176570 A | 06 Jul. 2006 | (Family: none) | |
| WO 206/123561 A1 | 23 Nov. 2006 | US 2008/0280154 A1 examples EP 1882701 A1 | |
| WO 2012/144564 A1 | 26 Oct. 2012 | US 2014/0031203 A1 examples EP 2700663 A1 CN 103502287 A | |
| WO 2016/006135 A1 | 14 Jan. 2016 | US 2016/0030919 A1 examples EP 2993189 A1 CN 104918964 A KR 10-2016-0017648 A | |
| JP 2016-28113 A | 25 Feb. 2016 | US 2017/0210831 A1 examples EP 3153528 A1 KR 10-2016-0142416 A CN 106471013 A | |
| JP 2016-28117 A | 25 Feb. 2016 | US 2017/0107313 A1 examples EP 2993191 A1 KR 10-2016-0017649 A CN 105517660 A | |

Form PCT/ISA/210 (patent family annex) (January 2015)

**EP 3 936 538 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H06345819 A
- JP H09510889 W